# EUROPÄISCHE PATENTSCHRIFT

(11) **EP 1 291 073 B1**
(45) Veröffentlichungstag und Bekanntmachung des Hinweises auf die Patenterteilung: **23.03.2005**
(21) Anmeldenummer: 01121542.3
(22) Anmeldetag: 10.09.2001
(51) Int. Cl.: B01J 13/16, B01J 13/20

(54) **Polyamidmikrokapseln**
Polyamide Microcapsules
Micrcapsules de Polyamide

(43) Veröffentlichungstag der Anmeldung: 12.03.2003
(73) Patentinhaber: Cognis Iberia, S.L., 08755 Castellbisbal, (Barcelona) (ES)
(72) Erfinder: Viladot Petit, Josep-Liuis, 08018 Barcelona (ES); De Moragas, Maria, Dr., 08310 Argentona Barcelona (ES)
(74) Vertreter: Fabry, Bernd, Dr.

(56) Entgegenhaltungen:
- US-A- 3 396 116
- US-A- 3 577 515
- US-A- 4 518 547

## Beschreibung

### Gebiet der Erfindung

Die Erfindung befindet sich auf dem Gebiet der Mikrokapseln und betrifft neue Systeme auf Polyamidbasis, die sich insbesondere dadurch auszeichnen, dass sie gegenüber Hypochloritlauge beständig sind, ein Verfahren zu ihrer Herstellung sowie ihre Verwendung für verschiedene Einsatzgebiete.

### Stand der Technik

Unter dem Begriff "Mikrokapsel" werden vom Fachmann sphärische Aggregate mit einem Durchmesser im Bereich von etwa 0,1 bis etwa 5 mm verstanden, die mindestens einen festen oder flüssigen Kern enthalten, der von mindestens einer kontinuierlichen Hülle umschlossen ist. Genauer gesagt handelt es sich um mit filmbildenden Polymeren umhüllte feindisperse flüssige oder feste Phasen, bei deren Herstellung sich die Polymere nach Emulgierung und Koazervation oder Grenzflächenpolymerisation auf dem einzuhüllenden Material niederschlagen. Nach einem anderen Verfahren werden geschmolzene Wachse in einer Matrix aufgenommen ("microsponge"), die als Mikropartikel zusätzlich mit filmbildenden Polymeren umhüllt sein können. Die mikroskopisch kleinen Kapseln lassen sich wie Pulver trocknen. Neben einkernigen Mikrokapseln sind auch mehrkernige Aggregate, auch Mikrosphären genannt, bekannt, die zwei oder mehr Kerne im kontinuierlichen Hüllmaterial verteilt enthalten. Ein- oder mehrkernige Mikrokapseln können zudem von einer zusätzlichen zweiten, dritten etc. Hülle umschlossen sein. Die Hülle kann aus natürlichen, halbsynthetischen oder synthetischen Materialien bestehen. Natürlich Hüllmaterialien sind beispielsweise Gummi Arabicum, Agar-Agar, Agarose, Maltodextrine, Alginsäure bzw. ihre Salze, z.B. Natrium- oder Calciumalginat, Fette und Fettsäuren, Cetylalkohol, Collagen, Chitosan, Lecithine, Gelatine, Albumin, Schellack, Polysaccaride, wie Stärke oder Dextran, Polypeptide, Proteinhydrolysate, Sucrose und Wachse. Halbsynthetische Hüllmaterialien sind unter anderem chemisch modifizierte Cellulosen, insbesondere Celluloseester und -ether, z.B. Celluloseacetat, Ethylcellulose, Hydroxypropylcellulose, Hydroxypropylmethylcellulose und Carboxymethylcellulose, sowie Stärkederivate, insbesondere Stärkeether und -ester. Synthetische Hüllmaterialien sind beispielsweise Polymere wie Polyacrylate, Polyamide, Polyvinylalkohol oder Polyvinylpyrrolidon.

Beispiele für Mikrokapseln des Stands der Technik sind folgende Handelsprodukte (in Klammern angegeben ist jeweils das Hüllmaterial) : *Hallcrest Microcapsules* (Gelatine, Gummi Arabicum), *Coletica Thalaspheres* (maritimes Collagen), *Lipotec Millicapseln* (Alginsäure, Agar-Agar), *Induchem Unispheres* (Lactose, mikrokristalline Cellulose, Hydroxypropylmethylcellulose); *Unicerin C30* (Lactose, mikrokristalline Cellulose, Hydroxypropylmethylcellulose), *Kobo Glycospheres* (modifizierte Stärke, Fettsäureester, Phospholipide), *Softspheres* (modifiziertes Agar-Agar) und *Kuhs Probiol Nanospheres* (Phospholipide).

In diesem Zusammenhang sei auch auf die deutsche Patentanmeldung **DE 19712978 A1** (Henkel) hingewiesen, aus der Chitosanmikrosphären bekannt sind, die man erhält, indem man Chitosane oder Chitosanderivate mit Ölkörpern vermischt und diese Mischungen in alkalisch eingestellte Tensidlösungen einbringt. Aus der deutschen Patentanmeldung **DE 19756452 A1** (Henkel) ist ferner auch die Verwendung von Chitosan als Verkapselungsmaterial für Tocopherol bekannt. Chitosanmikrokapseln und Verfahren zu ihrer Herstellung sind Gegenstand früherer Patenanmeldungen der Patentanmelderin **[WO 01/01926, WO 01/01927, WO 01/01928, WO 01/01929].** Man unterscheidet dabei im wesentlichen die beiden folgenden Verfahren:
(1) Mikrokapseln mit mittleren Durchmessern im Bereich von 0,1 bis 5 mm, bestehend aus einer Hüllmembran und einer mindestens ein Wachs enthaltenden Matrix, erhältlich, indem man
   (a) aus Gelbildnern, Chitosanen und Wirkstoffen eine Matrix zubereitet,
   (b) gegebenenfalls die Matrix in einer Ölphase dispergiert,
   (c) die dispergierte Matrix mit wässrigen Lösungen anionischer Polymeren vom Typ der Polyalginate oder anionischen Chitosanderivate behandelt und gegebenenfalls dabei die Ölphase entfernt.
(2) Mikrokapseln mit mittleren Durchmessern im Bereich von 0,1 bis 5 mm, bestehend aus einer Hüllmembran und einer mindestens ein Wachs enthaltenden Matrix, erhältlich, indem man
   (a) aus Gelbildnern, anionischen Polymeren vom Typ der Polyalginate oder anionischen Chitosanderivate und Wirkstoffen eine Matrix zubereitet,
   (b) gegebenenfalls die Matrix in einer Ölphase dispergiert,
   (c) die dispergierte Matrix mit wässrigen Chitosanlösungen behandelt und gegebenenfalls dabei die Ölphase entfernt.

Von Nachteil ist jedoch, dass die Mikrokapseln des Stands der Technik gegenüber aggressiven Medien nur sehr bedingt stabil sind. Kapseln des oben genannten Typs lösen sich beispielsweise in Hypochloritlösungen oder Wasserstoffperoxid schlicht auf, so dass sie mit diesen Stoffen nicht in Kontakt gebracht werden können. Andererseits ist es gerade für den amerikanischen und den südeuropäischen Raum, in dem Einsatz von solchen Mitteln für die Reinigung harter Oberflächen sehr verbreitet ist, wünschenswert, Mikrokapseln zur Verfügung stellen zu können, die sich in diesen Medien als hinreichend stabil erweisen und die darin enthaltenen Wirkstoffe erst nach Zuführung mechanischer Energie freisetzen.

Die Aufgabe der vorliegenden Erfindung hat folglich darin bestanden, neue Mikrokapseln zur Verfügung zu stellen, die in hypochlorit- bzw. wasserstoffperoxidhaltigen Lösungen stabil sind, d.h. sich über einen Zeitraum von wenigstens 4 Wochen nicht auflösen oder den sie enthaltenen Wirkstoff anders freisetzen.

### Beschreibung der Erfindung

Gegenstand der Erfindung sind Polyamidmikrokapseln mit einem mittleren Durchmesser im Bereich von 0,1 bis 5 mm, dadurch erhältlich, dass man
(a) eine wässrige Phase herstellt, in der (a1) Diamine und (a2) anionische Polymere und/oder Heteropolysaccharide gelöst sind,
(b) eine Ölphase herstellt, in der (b1) Dicarbonsäurehalogenide gelöst sind,
(c) die beiden Phasen unter Ausbildung einer W/O-Emulsion in Kontakt bringt, so dass an den Grenzflächen eine Polykondensationsreaktion unter Bildung von Polyamidmikrokapseln stattfindet,
(d) die Polyamidmikrokapseln abfiltriert und wäscht,
(e) die so aufgereinigten Polyamidmikrokapseln mit einer Calciumsalzlösung behandelt und dabei härtet, und schließlich
(f) die gehärteten Polyamidmikrokapseln erneut abfiltriert, wäscht und trocknet.

Überraschenderweise wurde gefunden, dass sich gerade Polyamidkapseln durch eine besondere Beständigkeit in Hypochloritlaugen und peroxidischen Mitteln auszeichnen, wozu es jedoch erforderlich ist, nach der Grenzflächenpolykondensation eine Aushärtung, d.h. Ausbildung von Verzweigungen in der Kapselhülle durch Calciumionen durchzuführen. Die so erhaltenen sphärischen Gebilde haben einen Durchmesser im sichtbaren Bereich und sind über einen Zeitraum von wenigstens 4 Wochen in gängigen hypochlorithaltigen Haushaltsreinigern beständig.

### Verfahren

Ein weiterer Gegenstand der Erfindung betrifft ein Verfahren zur Herstellung von Polyamidmikrokapseln mit einem mittleren Durchmesser im Bereich von 0,1 bis 5 mm, bei dem man
(a) eine wässrige Phase herstellt, in der (a1) Diamine und (a2) anionische Polymere und/oder Heteropolysaccharide gelöst sind,
(b) eine Ölphase herstellt, in der (b1) Dicarbonsäurehalogenide gelöst sind,
(c) die beiden Phasen unter Ausbildung einer W/O-Emulsion in Kontakt bringt, so dass an den Grenzflächen eine Polykondensationsreaktion unter Bildung von Polyamidmikrokapseln stattfindet,
(d) die Polyamidmikrokapseln abfiltriert und wäscht,
(e) die so aufgereinigten Polyamidmikrokapseln mit einer Calciumsalzlösung behandelt und dabei härtet, und schließlich
(f) die gehärteten Polyamidmikrokapseln erneut abfiltriert, wäscht und trocknet.

### Wässrige Phase

In einem ersten Schritt des erfindungsgemäßen Verfahrens wird eine wässrige Zubereitung hergestellt, welche als Komponente (a1) das für die Polykondensation benötigte Diamin enthält. Die Auswahl dieser Komponente ist wenig kritisch, demnach kommen grundsätzlich alle aliphatischen oder aromatischen Diamine in Betracht, die über eine hinreichende Wasserlöslichkeit verfügen. Besonders bevorzugt ist indes das für Nylon-6,6 bekannte Hexylendiamin.

Als weitere Komponente wird für die Ausbildung der Kapselhülle entweder ein Anionpolymer oder ein Heteropolysaccharid benötigt. Als Anionpolymere eignen sich insbesondere Alginsäuren und deren Salze. Bei der Alginsäure handelt es sich um ein Gemisch carboxylgruppenhaltiger Polysaccharide mit folgendem idealisierten Monomerbaustein:

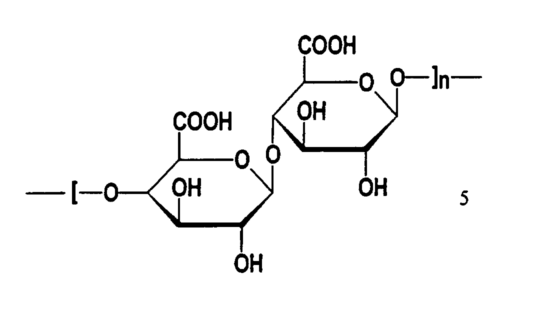

Das durchschnittliche Molekulargewicht der Alginsäuren bzw. der Alginate liegt im Bereich von 150.000 bis 250.000. Dabei sind als Salze der Alginsäure sowohl deren vollständige als auch deren partiellen Neutralisationsprodukte zu verstehen, insbesondere die Alkalisalze und hierunter vorzugsweise das Natriumalginat ("Algin") sowie die Ammonium- und Erdalkalisalze. besonders bevorzugt sind Mischalginate, wie z.B. Natrium/Magnesium- oder Natrium/Calciumalginate.

Als thermogelierende Heteropolysaccharide kommen vorzugsweise Agarosen in Frage, welche in Form des aus Rotalgen zu gewinnenden Agar-Agar auch zusammen mit bis zu 30 Gew.-% nicht-gelbildenden Agaropektinen vorliegen können. Hauptbestandteil der Agarosen sind lineare Polysaccharide aus D-Galaktose und 3,6-Anhydro-L-galaktose, die alternierend β-1,3- und β-1,4-glykosidisch verknüpft sind. Die Heteropolysaccharide besitzen vorzugsweise ein Molekulargewicht im Bereich von 110.000 bis 160.000 und sind sowohl farb- als auch geschmacklos. Als Alternativen kommen Pektine, Xanthane (auch Xanthan Gum) sowie deren Mischungen in Frage. Es sind weiterhin solche Typen bevorzugt, die noch in 1-Gew.-%iger wässriger Lösung Gele bilden, die nicht unterhalb von 80 °C schmelzen und sich bereits oberhalb von 40 °C wieder verfestigen.

Die wässrige Phase enthält die Komponenten (a) üblicherweise in Mengen von jeweils 0,1 bis 1, vorzugsweise 0,3 bis 0,8 Gew.-%. Des weiteren empfiehlt es sich, alle Wirkstoffe, die in den fertigen Kapseln enthalten sein sollen, wie beispielsweise Farbstoffe, Parfümstoffe, Konservierungsmittel und dergleichen, ebenfalls in die wässrige Phase einzubringen.

### Ölphase

Im zweiten Verfahrensschritt wird die Ölphase hergestellt, welche die zweite Kondensationskomponente, nämlich das Dicarbonsäurechlorid enthält. Auch hier ist die Auswahl der Dicarbonsäurekomponente wenig kritisch, im Hinblick auf die Herstellung von Nylon-6,6-Kapseln empfiehlt sich natürlich wieder Sebacinsäurechlorid. Alternativ können aber auch längerkettige Dicarbonsäuren, wie die bei der Fermentation von entsprechenden Paraffinen anfallende 1,18-Hexadecandicarbonsäure bzw. deren Chlorid eingesetzt werden. Auch die Auswahl des Öls ist wenig kritisch. In der Regel wird man Mineralöl ("Weißöl") bzw. Paraffinöl einsetzen, ebenfalls geeignet sind auch Siliconöle oder Esteröle sowie alle übrigen gängigen kosmetischen Ölkörper. Die Ölphase enthält die Dicarbonsäurechloride üblicherweise in Mengen von 1 bis 5 und vorzugsweise 2 bis 3 Gew.-%.

### Emulsionsbildung, Polykondensation, Härtung und Aufarbeitung

Im dritten Schritt findet die eigentliche Phasengrenzflächenpolykondensation statt. Hierzu werden die wässrige Phase und die Ölphase unter starker Scherung mit einander vermischt, wobei eine W/O-Emulsion erhalten wird. Typischerweise setzt man die beiden Phasen im Gewichtsverhältnis 1: 1 bis 1 : 3 und vorzugsweise etwa 1 : 2 ein. An den Grenzflächen der Wassertröpfchen zu der sie umgebenden Ölphase findet eine Polykondensation zwischen den Diaminen und den Dicarbonsäurechloriden statt und es bilden sich sphärische Gebilde mit einer Polyamidhülle und einer wässrigen inneren Phase, in der die Wirkstoffe enthalten sind. Diese Mikrokapseln sind noch sehr empfindlich und werden vorsichtig abfiltriert und mit Wasser oder verdünnter Tensidlösung gewaschen, um anhaftende Reste der Ölphase zu entfernen. Zur Härtung der so erhaltenen Mikrokapseln ist es erforderlich, die Polyamide zu verknüpfen ("cross-linken"), was vorzugsweise durch Einbringen von Calciumionen geschieht. Hierzu werden die Mikrokapseln mit wässrigen Calciumsalzlösungen, vorzugsweise einer 0,5 Gew.-%igen Calciumchloridlösung gewaschen. Auf 100 g Kapseln setzt man in der Regel 100 g einer 0,5 Gew.-%igen CaCl₂-Lösung ein. Die so gehärteten Kapseln werden nun abermals abfiltriert, gewaschen und getrocknet.

### Gewerbliche Anwendbarkeit

Ein weiterer Gegenstand der Erfindung betrifft die Verwendung der neuen Polyamidmikrokapseln zur Herstellung von hypochlorithaltigen Zubereitungen, speziell von Mitteln zur Reinigung harter Oberflächen, welche die Kapseln in Mengen von 1 bis 10 Gew.-% - bezogen auf die Mittel - enthalten können. Gleichwohl eignen sich die Mikrokapseln auch für andere Anwendungszwecke, beispielsweise zur Herstellung von kosmetischen Zubereitungen und zur Ausrüstung von textilen Flächengebilden.

### Beispiele

**Beispiel 1.** In einem 1-I-Dreihalskolben mit Rührer und Tropftrichter wurden bei Raumtemperatur 0,3 g Hexylendiamin, 0,1 g Alginsäure, 0,05 g Phenonip und 0,05 g Indanthrenblau RS (C.I. 69800) vorgelegt und mit destilliertem Wasser auf 100 ml aufgefüllt. Zu der Lösung wurde unter starkem Rühren innerhalb von 3 min 100 g einer 5 Gew.-%igen Lösung von Sebacinsäurechlorid in Paraffinöl getropft. Nach etwa 30 min war die Polykondensation abgeschlossen und die erhaltenen Polyamidmikrokapseln wurden vorsichtig über einem Büchnerfilter von der Ölphase abgetrennt und mit einer 3 Gew.-% Laurylsulfatlösung gewaschen, bis keine Ölreste mehr zu erkennen waren. Die Kapseln wurden in eine frische Rührapparatur überführt und dort mit einer 5 Gew.-% Calciumchloridlösung behandelt, wobei die Rührgeschwindigkeit so bemessen war, dass einerseits eine ausreichende Durchmischung stattfand, die noch nicht ausgehärteten Kapseln jedoch nicht zerschlagen wurden. Nach weiteren 30 min war die Härtung der Kapseln abgeschlossen. Diese wurden abermals abfiltriert, gewaschen und getrocknet. Der mittlere Durchmesser der Kapseln betrug 1 mm.

**Beispiel 2**. In einem 1-I-Dreihalskolben mit Rührer und Tropftrichter wurden bei Raumtemperatur 0,3 g Hexylendiamin, 0,2 g Agar Agar, 0,05 g Phenonip und 0,05 g Krapplack (C.I.58000) vorgelegt und mit destilliertem Wasser auf 100 ml aufgefüllt. Zu der Lösung wurde unter starkem Rühren innerhalb von 3 min 100 g einer 5 Gew.-%igen Lösung von Sebacinsäurechlorid in Paraffinöl getropft. Nach etwa 30 min war die Polykondensation abgeschlossen und die erhaltenen Polyamidmikrokapseln wurden vorsichtig über einem Büchnerfilter von der Ölphase abgetrennt und mit einer 3 Gew.-% Laurylsulfatlösung gewaschen, bis keine Ölreste mehr zu erkennen waren. Die Kapseln wurden in eine frische Rührapparatur überführt und dort mit einer 5 Gew.-% Calciumchloridlösung behandelt, wobei die Rührgeschwindigkeit so bemessen war, dass einerseits eine ausreichende Durchmischung stattfand, die noch nicht ausgehärteten Kapseln jedoch nicht zerschlagen wurden. Nach weiteren 30 min war die Härtung der Kapseln abgeschlossen. Diese wurden abermals abfiltriert, gewaschen und getrocknet. Der mittlere Durchmesser der Kapseln betrug 1 mm.

**Beispiel 3.** In einem 1-I-Dreihalskolben mit Rührer und Tropftrichter wurden bei Raumtemperatur 0,3 g Hexylendiamin, 0,1 g Alginsäure, 0,05 g Phenonip und 0,1 g Retinol vorgelegt und mit destilliertem Wasser auf 100 ml aufgefüllt. Zu der Lösung wurde unter starkem Rühren innerhalb von 3 min 100 g einer 3 Gew.-%igen Lösung von 1,18-Hexadecandicarbonsäurechlorid in Paraffinöl getropft. Nach etwa 30 min war die Polykondensation abgeschlossen und die erhaltenen Polyamidmikrokapseln wurden vorsichtig über einem Büchnerfilter von der Ölphase abgetrennt und mit einer 3 Gew.-% Laurylsulfatlösung gewaschen, bis keine Ölreste mehr zu erkennen waren. Die Kapseln wurden in eine frische Rührapparatur überführt und dort mit einer 5 Gew.-% Calciumchloridlösung behandelt, wobei die Rührgeschwindigkeit so bemessen war, dass einerseits eine ausreichende Durchmischung stattfand, die noch nicht ausgehärteten Kapseln jedoch nicht zerschlagen wurden. Nach weiteren 30 min war die Härtung der Kapseln abgeschlossen. Diese wurden abermals abfiltriert, gewaschen und getrocknet. Der mittlere Durchmesser der Kapseln betrug 1 mm.

**Beispiel 4.** Die gemäß Beispiele 1 bis 3 erhaltenen Polyamidmikrokapseln wurden in eine 5 Gew.-%ige Natriumhydroxidlösung eingebracht und dort über einen Zeitraum von 4 Wochen bei 30 °C gelagert. Alle Systeme erwiesen sich dabei als stabil.

## Patentansprüche

1. Polyamidmikrokapseln mit einem mittleren Durchmesser im Bereich von 0,1 bis 5 mm, dadurch erhältlich, dass man
(a) eine wässrige Phase herstellt, in der (a1) Diamine und (a2) anionische Polymere und/oder Heteropolysaccharide gelöst sind,
(b) eine Ölphase herstellt, in der (b1) Dicarbonsäurehalogenide gelöst sind,
(c) die beiden Phasen unter Ausbildung einer W/O-Emulsion in Kontakt bringt, so dass an den Grenzflächen eine Polykondensationsreaktion unter Bildung von Polyamidmikrokapseln stattfindet,
(d) die Polyamidmikrokapseln abfiltriert und wäscht,
(e) die so aufgereinigten Polyamidmikrokapseln mit einer Calciumsalzlösung behandelt und dabei härtet, und schließlich
(f) die gehärteten Polyamidmikrokapseln erneut abfiltriert, wäscht und trocknet.

2. Verfahren zur Herstellung von Polyamidmikrokapseln mit einem mittleren Durchmesser im Bereich von 0,1 bis 5 mm, bei dem man
(a) eine wässrige Phase herstellt, in der (a1) Diamine und (a2) anionische Polymere und/oder Heteropolysaccharide gelöst sind,
(b) eine Ölphase herstellt, in der (b1) Dicarbonsäurehalogenide gelöst sind,
(c) die beiden Phasen unter Ausbildung einer W/O-Emulsion in Kontakt bringt, so dass an den Grenzflächen eine Polykondensationsreaktion unter Bildung von Polyamidmikrokapseln stattfindet,
(d) die Polyamidmikrokapseln abfiltriert und wäscht,
(e) die so aufgereinigten Polyamidmikrokapseln mit einer Calciumsalzlösung behandelt und dabei härtet, und schließlich
(f) die gehärteten Polyamidmikrokapseln erneut abfiltriert, wäscht und trocknet.

3. Verfahren nach Anspruch 2, **dadurch gekennzeichnet, dass** man als Komponente (a1) Hexylendiamin einsetzt.

4. Verfahren nach den Ansprüchen 2 und/oder 3, **dadurch gekennzeichnet, dass** man als Komponente (a2) Alginsäure oder deren Salze einsetzt.

5. Verfahren nach mindestens einem der Ansprüche 2 bis 4, **dadurch gekennzeichnet, dass** man als Komponente (a2) Agarosen einsetzt.

6. Verfahren nach mindestens einem der Ansprüche 2 bis 5, **dadurch gekennzeichnet, dass** man wässrige Phasen einsetzt, die die Komponenten (a) in Mengen von jeweils 0,1 bis 1 Gew.-% enthalten.

7. Verfahren nach mindestens einem der Ansprüche 2 bis 6, **dadurch gekennzeichnet, dass** man als Komponente (b1) Sebacinsäurechlorid einsetzt.

8. Verfahren nach mindestens einem der Ansprüche 2 bis 7, **dadurch gekennzeichnet, dass** man Ölphasen einsetzt, die die Komponente (b) in Mengen von 1 bis 5 Gew.-% enthält.

9. Verfahren nach mindestens einem der Ansprüche 2 bis 8, **dadurch gekennzeichnet, dass** man die wässrige Phase und die Ölphase im Gewichtsverhältnis 1 : 1 bis 1 : 3 einsetzt.

10. Verfahren nach mindestens einem der Ansprüche 2 bis 9, **dadurch gekennzeichnet, dass** man die Polyamidmikrokapseln mit wässriger Calciumchloridlösung behandelt.

11. Verwendung von Polyamidmikrokapseln gemäß dem Anspruch 1 oder hergestellt gemäß den Anspruchen 2-10 zur Herstellung von hypochlorithaltigen Zubereitungen.

12. Verwendung von Polyamidmikrokapseln gemäß dem Anspruch 1 oder hergestellt gemäß den Anspruchen 2-10 zur Herstellung von kosmetischen Zubereitungen.

13. Verwendung von Polyamidmikrokapseln gemäß dem Anspruch 1 oder hergestellt gemäß den Anspruchen 2-10 zur Ausrüstung von textilen Flächengebilden.

## Claims

1. Polyamide microcapsules with a mean diameter of 0.1 to 5 mm obtainable by
(a) preparing an aqueous phase in which (a1) diamines and (a2) anionic polymers and/or heteropolysaccharides are dissolved,
(b) preparing an oil phase in which (b1) dicarboxylic acid halides are dissolved,
(c) contacting the two phases to form a w/o emulsion, so that a polycondensation reaction in which polyamide microcapsules are formed takes place at the interfaces,
(d) filtering off and washing the polyamide microcapsules,
(e) treating the polyamide microcapsules thus purified with a calcium salt solution and hardening them in the process and, finally,
(f) re-filtering off, washing and drying the hardened polyamide microcapsules.

2. A process for the production of polyamide microcapsules with a mean diameter of 0.1 to 5 mm, **characterized in that** it comprises the steps of
(a) preparing an aqueous phase in which (a1) diamines and (a2) anionic polymers and/or heteropolysaccharides are dissolved,
(b) preparing an oil phase in which (b1) dicarboxylic acid halides are dissolved,
(c) contacting the two phases to form a w/o emulsion, so that a polycondensation reaction in which polyamide microcapsules are formed takes place at the interfaces,
(d) filtering off and washing the polyamide microcapsules,
(e) treating the polyamide microcapsules thus purified with a calcium salt solution and hardening them in the process and, finally,
(f) re-filtering off, washing and drying the hardened polyamide microcapsules.

3. A process as claimed in claim 2, **characterized in that** hexylenediamine is used as component (a1).

4. A process as claimed in claims 2 and/or 3, **characterized in that** alginic acid or an alginic acid salt is used as component (a2).

5. A process as claimed in at least one of claims 2 to 4, **characterized in that** agaroses are used as component (a2).

6. A process as claimed in at least one of claims 2 to 5, **characterized in that** aqueous phases containing each component (a) in a quantity of 0.1 to 1% by weight are used.

7. A process as claimed in at least one of claims 2 to 6, **characterized in that** sebacic acid chloride is used as component (b1).

8. A process as claimed in at least one of claims 2 to 7, **characterized in that** oil phases containing component (b) in a quantity of 1 to 5% by weight are used.

9. A process as claimed in at least one of claims 2 to 8, **characterized in that** the aqueous phase and the oil phase are used in a ratio by weight of 1:1 to 1:3.

10. A process as claimed in at least one of claims 2 to 9, **characterized in that** the polyamide microcapsules are treated with aqueous calcium chloride solution.

11. The use of the polyamide microcapsules claimed in claim 1 or produced by the process claimed in claims 2 to 10 for the production of hypochlorite-containing preparations.

12. The use of the polyamide microcapsules claimed in claim 1 or produced by the process claimed in claims 2 to 10 for the production of cosmetic preparations.

13. The use of the polyamide microcapsules claimed in claim 1 or produced by the process claimed in claims 2 to 10 for finishing sheet-form textiles.

## Revendications

1. Microcapsules de polyamide ayant un diamètre moyen allant de 0,1 à 5 mm, que l'on peut obtenir
(a) en préparant une phase aqueuse dans laquelle sont dissous (a1) des diamines et (a2) des polymères anioniques et/ou des hétéropolysaccharides,
(b) en préparant une phase huileuse dans laquelle sont dissous (b1) des halogénures d'acides dicarboxyliques,
(c) en mettant les deux phases en contact avec formation d'une émulsion E/H (eau-dans-huile), de manière qu'une réaction de polycondensation avec formation de microcapsules de polyamide, se produise sur les interfaces,
(d) en filtrant et en lavant les microcapsules de polyamide,
(e) en traitant les microcapsules de polyamide ainsi purifiées avec une solution de sel de calcium de manière à les durcir, et enfin
(f) en filtrant à nouveau les microcapsules de polyamide durcies, en les lavant et en les séchant.

2. Procédé de fabrication de microcapsules de polyamide ayant un diamètre moyen allant de 0,1 à 5 mm, selon lequel
(a) on prépare une phase aqueuse, dans laquelle sont dissous (a1) des diamines et (a2) des polymères anioniques et/ou des hétéropolysaccharides,
(b) on prépare une phase huileuse dans laquelle sont dissous (b1) des halogénures d'acides dicarboxyliques,
(c) on met les deux phases en contact avec formation d'une émulsion E/H (eau-dans-huile), de manière qu'une réaction de polycondensation avec formation de microcapsules de polyamide, se produise sur les interfaces,
(d) on filtre et on lave les microcapsules de polyamide,
(e) on traite les microcapsules de polyamide ainsi purifiées avec une solution de sel de calcium de manière à les durcir, et enfin
(f) on filtre à nouveau les microcapsules de polyamide durcies, on les lave et on les sèche.

3. Procédé selon la revendication 2,
**caractérisé en ce qu**
comme composant (a1) on utilise l'hexylènediamine.

4. Procédé selon les revendications 2 et/ou 3,
**caractérisé en ce que**
comme composant (a2) on utilise l'acide alginique ou ses sels.

5. Procédé selon au moins une des revendications 2 à 4,
**caractérisé en ce que**
comme composant (a2) on utilise des agaroses.

6. Procédé selon au moins une des revendications 2 à 5,
**caractérisé en ce que**
on utilise des phases aqueuses qui contiennent les composants (a) en quantités respectives de 0,1 à 1 % en poids.

7. Procédé selon au moins une des revendications 2 à 6,
**caractérisé en ce que**
comme composant (b1) on utilise le chlorure de l'acide sébacique.

8. Procédé selon au moins une des revendications 2 à 7,
**caractérisé en ce qu'**
on utilise des phases huileuses qui contiennent le composant (b) en quantités de 1 à 5 % en poids.

9. Procédé selon au moins une des revendications 2 à 8,
**caractérisé en ce qu'**
on utilise la phase aqueuse et la phase huileuse dans un rapport pondéral de 1 : 1 à 1 : 3.

10. Procédé selon au moins une des revendications 2 à 9,
**caractérisé en ce qu'**
on traite les microcapsules de polyamide avec une solution aqueuse de chlorure de calcium.

11. Utilisation de microcapsules de polyamide selon la revendication 1 ou fabriquées selon les revendications 2 à 10, pour produire des préparations contenant un hypochlorite.

12. Utilisation de microcapsules de polyamide selon la revendication 1 ou fabriquées selon les revendications 2 à 10, pour produire des préparations cosmétiques.

13. Utilisation de microcapsules de polyamide selon la revendication 1 ou fabriquées selon les revendications 2 à 10, pour apprêter des articles textiles en nappe.
